(19) **Europäisches Patentamt**

**European Patent Office**

**Office européen des brevets**

(11) **EP 1 288 657 B1**

(12) **EUROPÄISCHE PATENTSCHRIFT**

(45) Veröffentlichungstag und Bekanntmachung des Hinweises auf die Patenterteilung:
**30.11.2005 Patentblatt 2005/48**

(51) Int Cl.$^7$: **G01N 33/00**

(21) Anmeldenummer: **02012233.9**

(22) Anmeldetag: **04.06.2002**

(54) **Verfahren zum Bestimmen der Reduktionsmittelkonzentration (NH3) im Abgasstrom eines Verbrennungsmotors**

Method for determining the reducing agent concentration (NH3) in the exhaust gas stream of a combustion engine

Procédé de mesure de la concentration d'agent réducteur (NH3) dans le courant des gaz d'échappement d'un moteur à combustion

(84) Benannte Vertragsstaaten:
**DE ES FR GB IT SE**

(30) Priorität: **29.08.2001 DE 10142236**

(43) Veröffentlichungstag der Anmeldung:
**05.03.2003 Patentblatt 2003/10**

(73) Patentinhaber: **DaimlerChrysler AG
70567 Stuttgart (DE)**

(72) Erfinder:
• **Irion, Eckard
71111 Waldenbuch (DE)**
• **Knezevic, Aleksandar, Dr.
88045 Friedrichshafen (DE)**
• **Leye, Holger
72669 Unterensingen (DE)**
• **Smuk, Mirko
68753 Waghäusel (DE)**

(56) Entgegenhaltungen:
DE-C- 19 907 669        US-A- 4 565 679
US-A- 4 751 054         US-A- 4 829 440

• MOOS R. ET AL.: "Selective Ammonia Exhaust Gas Sensor for Automotive Applications" TRANSDUCERS '01. EUROSENSORS XV. 11TH INTERNATIONAL CONFERENCE ON SOLID-STATE SENSORS AND ACTUATORS. SPROINGER-VERLAG, Bd. 2, - 14. Juni 2001 (2001-06-14) Seiten 1684-1687, XP008027091 Germany ISBN: 3-540-42150-5
• MEIXNER H ET AL: "Thin-film gas sensors based on semiconducting metal oxides" SENSORS AND ACTUATORS B, ELSEVIER SEQUOIA S.A., LAUSANNE, CH, Bd. 23, Nr. 2/3, 1. Februar 1995 (1995-02-01), Seiten 119-125, XP004004800 ISSN: 0925-4005

## Beschreibung

Die Erfindung betrifft ein Verfahren zum Bestimmen der Reduktionsmittelkonzentration (NH3) im Abgasstrom eines Verbrennungsmotors nach dem Oberbegriff des Anspruchs 1.

Die Entstickung von dieselmotorischen Abgasen kann durch die selektive katalytische Reduktion (SCR) unter Verwendung von Ammoniak (NH3) als Reduktionsmittel durchgeführt werden. Dabei wird Ammoniak entweder direkt oder in Form einer Verbindung, aus der Ammoniak gewonnen wird, im Fahrzeug mitgeführt. Für die katalytische Reduktionsreaktion wird dem motorischen Abgas Ammoniak in einem bestimmten Verhältnis zum momentan im Abgas vorliegenden NOx-Gehalt zudosiert. Zum Erreichen des maximal möglichen NOx-Umsatzes muss ein stöchiometrisches NH3-NOx-Verhältnis vorhanden sein. Ein kleineres Verhältnis führt zu einem geringeren Umsatz, ein größeres Verhältnis führt zu einem so genannten "NH3-Durchbruch". Die optimale Ausnutzung des Entstickungskatalysators wird mit Hilfe eines zeolithischen NH3-Gassensors erreicht, der als Regelglied oder als NH3-Durchbruchssensor im Abgasstrom hinter dem Katalysator angebracht ist. Dann kann das NH3-NOx-Verhältnis immer auf den maximal möglichen NOx Umsatz eingestellt werden.

Für den Einsatz eines zeolithischen NH3-Gassensors ist es erforderlich, einen Zusammenhang zwischen Messgröße und der zu bestimmenden NH3-Konzentration am Sensor zu finden. Neben der Skalierung dient dieser Zusammenhang der rechnerischen Kompensation von unerwünschten Quereffekten, insbesondere denen gegenüber Wasser (H2O) in Form von Wasserdampf im Abgas.

Aus dem Konferenzbeitrag "Moos et al., Ammonia Exhaust Gas Sensor for Automotive Applications, Transducers '01 Eurosensors XV, 11th International Conference on Solid-State Sensors and Actuators, 10. - 14. Juni 2001, München, Springer-Verlag Bd. 2, S. 1684 - 1687, ISBN 3-540-42150-5" ist ein solcher für Automobilanwendungen geeigneter NH3-Sensor beschrieben. Der Sensor ist in Dickfilmtechnik gefertigt und weist eine hohe Empfindlichkeit gegenüber NH3, bei gleichzeitig sehr geringer Querempfindlichkeit gegenüber H2O und NO auf.

Aus der DE 199 07 669 C1 ist ein Verfahren zur Korrektur des Einflusses von H2O auf das Signal eines NH3-Gassensors bekannt. Dort wird vorgeschlagen, eine so genannte Nullwert-Funktion des Gassensors zu ermitteln, anhand derer die Messwerte im laufenden Betrieb korrigiert werden. Zur Bestimmung der Nullwert-Funktion wird bei laufendem Motor das Signal des Gassensors bei mehreren verschiedenen Motorbetriebsphasen gemessen, ohne dabei dem Abgas-Katalysatorsystem NH3 zuzuführen. Gleichzeitig wird die H2O-Konzentration des Abgases, das so genannte Feuchte-Äquivalent FÄ bestimmt. Das Feuchte-Äquivalent kennzeichnet den Betriebszustand des Motors. Es wird aus dem Kraftstoffmassenfluss zusammen mit dem Luftmassenstrom oder aus dem Sauerstoffpartialdruck des Abgases - jeweils auch in Kombination mit einem Feuchtesensor zum Bestimmen der H2O-Konzentration in der Ansaugluft - oder durch einen Feuchtesensor im Abgasstrom ermittelt. In allen Fällen wird aus den Messwerten die Steigung und der Achsenabschnitt der als Gerade angenäherten Nullwert-Funktion bestimmt. Zur Korrektur der Messwerte des NH3-Sensors während des Betriebs wird ausgehend vom Feuchte-Äquivalent des Motors der entsprechende Wert der Nullwert-Funktion ermittelt und der Messwert des Sensors entsprechend korrigiert.

Das bekannte Verfahren weist eine Reihe von Nachteilen auf.

Das verzögerte Ansprechen (Ansprechschwelle) des NH3 Sensors bei geringen NH3-Konzentrationen wird nicht berücksichtigt. Ebenso bleibt der Einfluss der NOx-Gasbestandteile auf die Ansprechschwelle und die NH3-Empfindlichkeit unberücksichtigt. Bei der Bestimmung der Nullwert-Funktion wird die Abhängigkeit zwischen Messgröße (Cp) und NH3-Konzentration linear angenähert, was lediglich für NH3-Konzentrationen bis ca. 50 ppm akteptable Ergebnisse liefert. Das Adsorptions- und Umsetzungsverhalten des NH3 Sensors, das zur Verfälschung der im Sensor eintreffenden Gaszusammensetzung führt, wird auch nicht berücksichtigt.

Aufgabe der Erfindung ist es, basierend auf einem NH3-Sensor ein Verfahren zum Bestimmen der Reduktionsmittelkonzentration (NH3) im Abgasstrom eines Verbrennungsmotors anzugeben, welches eine verbesserte Genauigkeit aufweist. Diese Aufgabe wird durch ein Verfahren mit den Merkmalen des Anspruchs 1 gelöst. Die Ausgestaltung des Verfahrens erfolgt gemäß den Merkmalen der abhängigen Ansprüche.

Bei einem Verfahren zum Bestimmen der Reduktionsmittelkonzentration (NH3) im Abgasstrom eines Verbrennungsmotors mit einem (zeolithischen) NH3 Gassensor, der einen Roh-Messwert liefert, wird der Roh-Messwert zunächst um einen OffsetWert und um einen von der H2O-Konzentration des Abgases abhängigen Korrekturwert zu einem Zwischenwert korrigiert. Anschließend wird der Zwischenwert um einen weiteren von der NOx-Konzentration des Abgases abhängigen Wert zum Messwert korrigiert.

Die Abhängigkeiten der Messwerte zur NOx- und NH3-Konzentration werden in einer 2-dimensionalen Tabelle abgelegt.

Zum Bestimmen der NH3-Konzentration werden die beiden benachbarten Spalten in der Tabelle mit der besten Übereinstimmung zur gegebenen NOx-Konzentration ausgewählt, dann wird in der ersten gewählten Spalte die Zeile mit der beste Übereinstimmung mit dem ersten Zwischenwert bestimmt und schließlich wird durch Interpolation zunächst zwischen den Spalten und dann den Zeilen der endgültige Messwert bestimmt.

Die Erfindung soll nachfolgend anhand von Ausführungsbeispielen und Figuren näher erläutert werden. Kurze Beschreibung der Figuren:

Figur 1    zeigt NOx-Korrektur der NH3-Konzentration am Sensor.

Figur 2    zeigt Vergleich Modelle zu Messdaten.

Figur 3    zeigt NH3-Sensor Ansprechverhalten im erweiterten Messbereich.

In den Betrachtungen nach dem eingangs erwähnten Stand der Technik wurde ein stark vereinfachtes Sensormodell unter folgenden Annahmen angewandt:

Die Parallelkapazität Cp im Ersatzschaltbild des Sensors charakterisiert NH3-Konzentration. Es gibt eine lineare Abhängigkeit zwischen NH3-Konzentration und Parallelkapazität Cp im Ersatzschaltbild des Sensors. H2O hat einen von der NH3 Konzentration unabhängigen, linearen Einfluss auf die Parallelkapazität Cp im Ersatzschaltbild des Sensors. Es gibt keinen Einfluss von Stickoxiden NOx auf das Sensorsignal. Und es finden keine Wechselwirkungen zwischen Gaskomponenten statt.

Daraus folgt für die gemessene Kapazität des Sensorelementes:

$$(1) \qquad C_p = C_{p0} + \nu_{NH3} \cdot NH3 + \nu_{H2O} \cdot H2O$$

$C_p$ : Gesamtkapazität Sensorelement
$C_{p0}$ : Grundkapazität
$V_{NH3}$ : Faktor NH3-Einfluß
$V_{H2O}$ : Faktor H2O-Einfluß
NH3 : Konzentration NH3
H2O : Konzentration H2O

Bezogen auf die gesuchte NH3-Konzentration und der Messgröße Cp ergibt sich:

$$(2) \qquad NH3_{Modell} = \nu'_{NH3} \cdot (C_p - C_{p0}) + \nu'_{H2O} \cdot H2O$$

Das Modells ist einfach im Ansatz und kommt mit 3 (Kalibrierungs-)Parameter plus 1 Messwert aus.
Die Nachteile des auf diesem Modell basierenden Korrekturverfahren sind:

- Verzögertes Ansprechen (Ansprechschwelle) bei geringen NH3-Konzentrationen wird nicht berücksichtigt
- Einfluss der NOx-Gasbestandteile auf Ansprechschwelle und NH3-Empfindlichkeit wird nicht berücksichtigt
- Experimentell ermittelte nicht-lineare Abhängigkeit zwischen Messgröße (Cp) und NH3-Konzentration wird linear angenähert, was lediglich für NH3-Konzentration bis ca. 50ppm akzeptable Ergebnisse liefert
- Adsorptions- und Umsetzungsverhalten des Sensors/Packaging, die zur Verfälschung der im Sensor eintreffenden Gaszusammensetzung führen werden nicht berücksichtigt.

Das auf diesem einfachen Modell basierenden Korrekturverfahren genügt des Erfordernissen im Abgassystem und den dort zu erwartenden Umgebungsbedingungen im Hinblick auf die Messgenauigkeit nicht. Dabei setzt sich der Messfehler aus drei Bestandteilen zusammen: den durch den Sensor bedingten, den Modellfehlern und den Fehlern in der Auswertungselektronik (Digitalisierungsfehler, Signalrauschen, ...).

$$(3) \qquad F\_ges = F\_Sensor + F\_Modell + F\_Elektronik$$

Insbesondere der Einfluss der Querempfindlichkeiten durch geänderte Umgebungsbedingen kann durch Verkleinerung des Modellfehlers deutlich verbessert werden.
Dem neuen Verfahren liegen folgende Erkenntnisse zugrunde:

- Im Bereich >20ppm existiert ein nahezu logarithmischer Zusammenhang zwischen der NH3-Konzentration und Cp.
- bei sehr niedrigen Konzentrationen (<20ppm) verzögert sich die Kapazitätszunahme, was durch die Anwesenheit von NOx noch verstärkt wird
- Auch bei höheren NH3-Konzentrationen zeigt sich eine verkleinerte Empfindlichkeit gegenüber NH3 bei Anwesenheit von Stickoxiden

- Stickoxide habe erst mit Anwesenheit von NH3 signifikanten Einfluss auf das Messergebnis (Wechselwirkung, Nebeneffekt)
- Einfluss des Wasser auf das Messergebnis ohne Wechselwirkung zum NH3-Effekt und im Bereich von 1-8vol% H2O linear

Ohne die Berücksichtigung der NOx-Effekte folgt somit:

$$(4) \qquad C_p = C_{p0} + v_{NH3} \cdot log(\frac{NH3}{S_{NH3}} + 1) + v_{H2O} \cdot H2O$$

$C_p$ : Gesamtkapazität Sensorelement
$C_{p0}$: Grundkapazität
$V_{NH3}$ : Faktor NH3-Einfluß (Empfindlichkeit)
NH3 : Konzentration NH3
$S_{NH3}$ : Faktor zur Charakterisierung des NH3-Einflusses
$V_{H2O}$ : Faktor zur Charakterisierung des H2O-Einflusses
H2O : Konzentration H2O

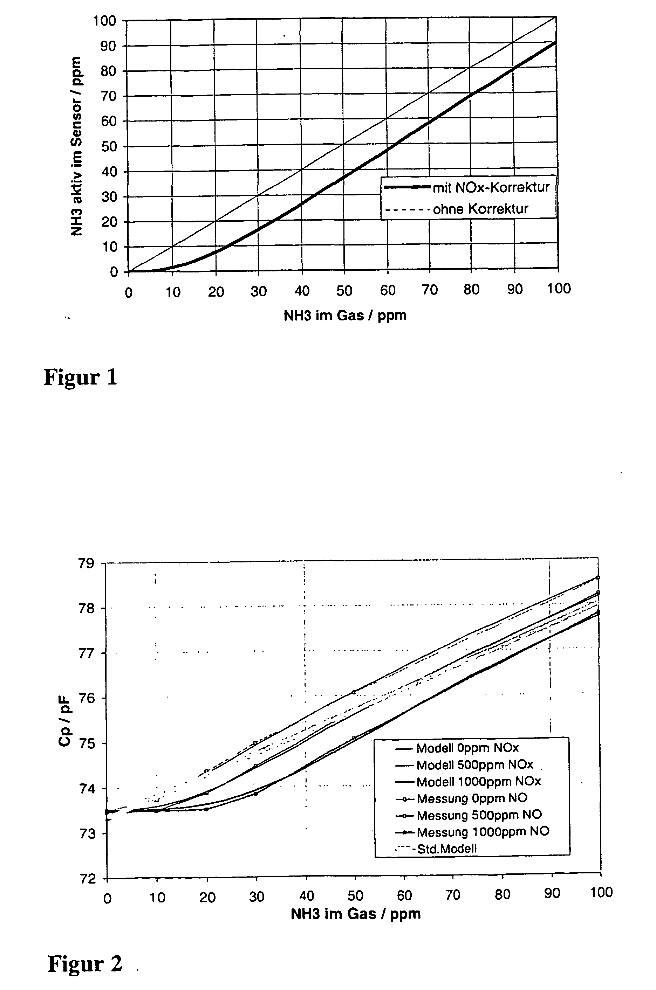

Durch die Anwesenheit der Stickoxide, katalytische Oberflächen, Adsorptionen und Zersetzungsvorgänge reagieren aber bereits ein Teil der NH3-Moleküle an dem Sensor und können von diesem nicht mehr wahrgenommen werden. Figur 1 zeigt exemplarisch den Unterschied zwischen NH3-Konzentration im Gas und der im Sensoren zum Messeffekt beitragenden.

Zur Kompensation dieses Einflusses wird der NH3-Term aus (4) durch den korrigierten ersetzt:

$$(5) \qquad NH3_{Sensor} = \frac{NH3^p}{NH3^p + \Delta} \; NH3 = \frac{NH3^{p+1}}{NH3^p + \Delta}$$

p : Einflussparameter NOx (Breite der Übergangszone)
NH3 : NH3-Konzentration im Gas
$NH3_{Sensor}$ : sensorisch wirksame NH3-Konzentration

Mit der Abhängigkeit

$$(6) \qquad \Delta = a_{NO} + v_{NO} \cdot NO$$

$a_{NO}$ : Einflussparameter NOx-unabhängiger Vorgänge (Adsorption, katalytische Umsetzung)
$v_{NO}$ : Einflussparameter NOx
NO : Konzentration der Stickoxide NOx

Im Grenzfall $\Delta \rightarrow 0$ oder bei sehr großer NH3-Konzentrationen geht (5) in $NH3_{korr} = NH3$ über.
Mit (4), (5) und (6) folgt für die korrigierte Gesamtkapazität Cp:

$$(7) \qquad C_p = C_{p0} + v_{NH3} \cdot log\left(\frac{NH3^{p+1} \cdot s_{NH3}}{NH3^p + a_{NO} + v_{NO} \cdot NO} + 1\right) + v_{H2O} \cdot H2O$$

Bereits im typische engen Arbeitsbereich des NH3-Sensors mit max. 100ppm NH3 und bis 1000pm NO zeigt das neue Verfahren seine Vorteile. Aus Figur 2 ist leicht zu erkennen, dass eine lineare Annäherung an die Messergebnisse deutlich schlechtere Aussagen liefern würde.
So ist bei in einem Einsatzbereich von

1-8 vol% H2O und

0-100ppm NH3 sowie
0/500/1000 ppm NOx
folgender maximaler Fehler zu erwarten

| $\Delta Cp/pF$ | Stand der Technik: | Erfindung: |
|---|---|---|
| 0ppm NO | 0,53 | 0,05 |
| 500ppm NO | 0,37 | 0,09 |
| 1000ppm NO | 0,87 | 0,11 |
| Max. | 0,87 (18ppm NH3) | 0,11 (2ppm NH3) |

Berücksichtigt man weiterhin, dass in diesem Fehler auch die Messfehler durch die verwendete Messgeräte und den Aufbau einfließen, kann man selbst in dem eingeschränkten Arbeitsbereich von einer Verbesserung um mindestens Faktor 8 ausgehen.

Insbesondere an/über den Grenzen des Arbeitsbereiches unter einer der folgenden Bedingungen

- Gaszusammensetzungen mit NOx-Konzentrationen >500ppm
- sehr kleine NH3-Konzentrationen <20 ppm
- große NH3-Konzentrationen >100 ppm

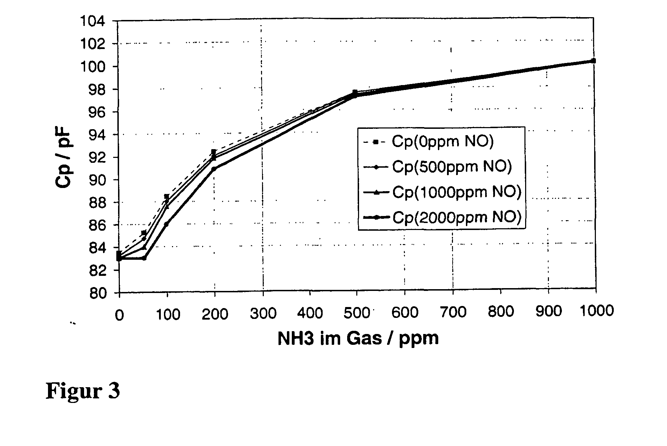

nimmt der Vorsprung in der Vorhersagegenauigkeit weiter zu. Dies ist an den in Figur 3 dargestellten Messergebnissen in dem erweiterten Bereich sehr gut zu erkennen.

Die deutliche Zunahme der Komplexität des Modells führt bedauerlicherweise dazu, dass Gleichung (7) nicht mehr algebraisch nach NH3 umgestellt werden kann und weiterhin der für die Signalverarbeitung bisher eingesetzte Mikrokontroller die notwendigen Berechnungen nicht mehr in der zur Verfügung stehenden Zeit ausführen könnte.

Der Ausweg über eine vollständige Tabellierung ist nicht praktikabel, da durch die 3 Einflussgrößen ein großer Speicher nötig wird. Zerlegt man Gleichung (7) in die drei Bestandteile

$$(8) \quad C_p = C_{p0} + C_p(NH_3NO_x) + C_p(H2O).$$

so wird ersichtlich, dass sich sowohl die Leerkapazität Cp0 als auch der H2O-Term durch Subtraktion eliminiert werden können. Die hierfür notwendigen 2 Parameter sind im nichtflüchtigen Speicher der Elektronik hinterlegt. Die H2O-Konzentration. wird aus externen Daten gewonnen. Die verbleibenden Abhängigkeiten zur NH3-und NO-Konzentration werden, wie in Tabelle 1 gezeigt, in einer 2-dimensionalen Tabelle, einer sogenannten Querempfindlichkeitstabelle, abgelegt.

Tabelle 1:

| Querempfindlichkeitstabelle | | | |
|---|---|---|---|
| $\Delta Cp/pF$ | NOx=0ppm | NOx=100ppm | NOx=200ppm |
| NH3=0 ppm | 0,0 | 0,0 | 0,0 |
| NH3=20 ppm | 1,1 | 1,0 | 0,9 |
| NH3=40 ppm | 1,6 | 1,5 | 1,4 |
| NH3=60 ppm | 2,0 | 1,9 | 1,8 |
| ... | ... | ... | ... |

Im Gesamtablauf wirkt der Algorithmus bereits recht früh ein:

- Sensorelementherstellung
- Abfahren von Eckdaten gemäß Rahmenbedingung des Algorithmus und Vermessung des Sensors an diesen Punkten
- Regression der Modellparameter mittels zuvor bestimmter Messdaten

- Bestimmung der 2 Parameter für Cp0 und H2O
- Berechnung der o.g. Wertetabellen mittels Algorithmus und Rahmenbedingungen des Einsatzgebietes
- Speicherung der Kenndaten und der Tabelle in den Speicher des Mikrokontroller
- Einsatz des Sensors unter Verwendung eines Interpolationsalgorithmus

Die Sensorelektronik geht dabei wie folgt vor um aus dem Messwert Cp die NH3-Konzentration zu bestimmen:

- Subtraktion der Leerkapazität vom Messwert mit gespeichertem Parameter Cp0.
- Subtraktion des H2O-Einflusses mit H2O-Parameter und gelieferter H2O-Konzentration.
- Die beiden benachbarten Spalten in der Querempfindlichkeitstabelle mit der besten Übereinstimmung zur gegebenen NOx-Konzentration werden ausgewählt.
- Durch Suchalgorithmus (z.B. Bisektionsverfahren) wird in ersten gewählter Spalte die Zeile mit der beste Übereinstimmung mit dem umgerechneten Messwert bestimmt.
- Durch lineare Interpolation zwischen zunächst Spalten und Zeilen wird durch Projektion auf die NH3-Zeilenzuordnung der NH3-Wert bestimmt.
- Ausgabe der ermittelten NH3-Konzentration.

Der komplette Algorithmus kann mit diesem Vorgehen durch elementare Operationen wie Addition und Multiplikation durchgeführt werden. Durch geeignete Wahl der Stützpunkte in der Tabelle kann der Fehler durch die lineare Interpolation sehr klein gehalten werden. Zweckmäßig ist es, die Stützpunktdichte in Bereichen hoher Nichtlinearität größer zu wählen.

**Patentansprüche**

1. Verfahren zum Bestimmen der Konzentration von als Reduktionsmittel verwendetem Ammoniak ($NH_3$) im Abgasstrom eines Verbrennungsmotors mit einem $NH_3$-Gassensor, bei welchem ein hinsichtlich einer Querempfindlichkeit des Gassensors gegenüber Wasser ($H_2O$) korrigierter $NH_3$-Messwert generiert wird,
**dadurch gekennzeichnet, dass**
das Verfahren folgende Schritte umfasst

   - Erfassen eines vom Gassensor bereitgestellten Rohmesswerts,
   - Generieren eines Zwischenwerts durch Korrektur des Rohmesswerts um einen Offsetwert und einen von der $H_2O$-Konzentration des Abgases abhängigen Korrekturwert und
   - Verändern des Zwischenwerts in Abhängigkeit von der $NO_x$-Konzentration des Abgases zum korrigierten $NH_3$-Messwert.

2. Verfahren nach Anspruch 1,
**dadurch gekennzeichnet, dass**
die Veränderung des Zwischenwerts in Abhängigkeit von der Größe des Zwischenwerts vorgenommen wird.

3. Verfahren nach Anspruch 1 oder 2,
**dadurch gekennzeichnet, dass** ein zeolithischer Gassensor verwendet wird.

4. Verfahren nach einem der Ansprüche 1 bis 3,
**dadurch gekennzeichnet, dass**
der Gassensor als kapazitiver Sensor ausgebildet ist.

5. Verfahren nach Anspruch 4,
**dadurch gekennzeichnet, dass**
als Offsetwert eine Grundkapazität des Gassensors verwendet wird.

**Claims**

1. Method for determining the concentration of ammonia ($NH_3$) used as the reducing agent in the exhaust gas stream of an internal combustion engine with an $NH_3$ gas sensor, in which a measurement value for $NH_3$ corrected in relation to transverse sensitivity towards water ($H_2O$) is generated,

**characterised in that**

the method comprises the following steps:

- detection of a raw measurement value provided by the gas sensor,
- generation of an intermediate value by correction of the raw measurement value by an offset value and a correction value that depends on the $H_2O$ concentration in the exhaust gas, and
- modification of the said intermediate value as a function of the $NO_x$ concentration in the exhaust gas to obtain the corrected $NH_3$ measurement value.

**2.** Method according to Claim 1,
**characterised in that**
the intermediate value is modified as a function of the magnitude of the said intermediate value.

**3.** Method according to Claims 1 or 2,
**characterised in that**
a zeolitic gas sensor is used.

**4.** Method according to any of Claims I to 3,
**characterised in that**
the gas sensor is made as a capacitative gas sensor.

**5.** Method according to Claim 4,
**characterised in that**
a fundamental capacity of the gas sensor is used as the offset value.

**Revendications**

**1.** Procédé de détermination de la concentration de l'ammoniac ($NH_3$), utilisé comme réducteur, dans le courant de gaz d'échappement d'un moteur à combustion interne, avec une sonde de gaz $NH_3$, dans lequel est engendrée une valeur de mesure de $NH_3$ corrigée du point de vue d'une sensibilité croisée de la sonde à l'eau ($H_2O$),
**caractérisé en ce que**
le procédé comprend les étapes suivantes :

- Saisie d'une valeur de mesure brute mise à disposition par la sonde de gaz
- Génération d'une valeur intermédiaire par correction de la valeur de mesure brute d'une valeur de décalage et d'une valeur de correction dépendante de la concentration du gaz d'échappement en $H_2O$ et
- Modification de la valeur intermédiaire en fonction de la concentration en $NO_x$ du gaz d'échappement pour donner la valeur $NH_3$ corrigée.

**2.** Procédé selon la revendication 1,
**caractérisé en ce que**
la modification de la valeur intermédiaire est effectuée en fonction de la grandeur de la valeur intermédiaire.

**3.** Procédé selon la revendication 1 ou 2,
**caractérisé en ce que** l'on utilise une sonde de gaz à zéolithe.

**4.** Procédé selon l'une des revendications 1 à 3,
**caractérisé en ce que**
la sonde de gaz est configurée comme sonde capacitive.

**5.** Procédé selon la revendication 4,
**caractérisé en ce que**
l'on utilise comme valeur de décalage une capacité de base de la sonde de gaz.

**Figur 1**

**Figur 2**

**Figur 3**